# EUROPEAN PATENT SPECIFICATION

(11) **EP 2 254 493 B1**
(45) Date of publication and mention of the grant of the patent: **02.03.2016**
(21) Application number: 09719020.1
(22) Date of filing: 02.03.2009
(51) Int. Cl.: A61B 17/80, A61B 17/86

(54) **VARIABLE AXIS LOCKING MECHANISM FOR USE IN ORTHOPEDIC IMPLANTS**
ARRETIERMECHANISMUS MIT VERÄNDERLICHER ACHSE FÜR ORTHOPÄDISCHE IMPLANTATE
MÉCANISME DE BLOCAGE À AXE VARIABLE DESTINÉ À ÊTRE UTILISÉ DANS DES IMPLANTS ORTHOPÉDIQUES

(30) Priority: 03.03.2008 US 67952 P; 26.02.2009 US 380339
(43) Date of publication of application: 01.12.2010
(73) Proprietor: Orthohelix Surgical Designs, Inc., Medina, OH 44256 (US)
(72) Inventor: LEWIS, Derek, Copley OH 44321 (US); KAY, David, B., Akron OH 44333 (US)
(74) Representative: Grand, Guillaume
(86) International application number: PCT/US2009/001359
(87) International publication number: WO 2009/114094

(56) References cited:
- FR-A1- 2 861 980
- US-A- 3 596 656
- US-A- 5 531 746
- US-A- 5 531 746
- US-A1- 2005 043 736
- US-A1- 2005 143 742
- US-A1- 2007 162 147
- US-B1- 6 235 033
- US-B2- 7 175 624

## Description

### FIELD OF THE INVENTION

This present invention relates to a mechanism for allowing a screw or peg to be used in an orthopedic implant, such as a plate, at a variable axis and subsequently to be locked into a desired orientation into a threaded opening.

### BACKGROUND OF THE INVENTION

US20051143742 A1 discloses a device which is used for solidly connecting a part such as a plate to an underlying support using at least one fixing element such as a screw. Said fixing element takes the form of a threaded rod a screw which passes through a hole housing a ring belonging to the part, such that it is screwed into the support material. The ring is a constriction ring comprising a non-circular outer profile which co-operates with the non-circular inner profile of the hole, which is in the part.

US 3,596,656 discloses a device for use in the fixation of the fracture of the bone comprising a metal plate having front and back faces and having a bore therethrough.

US 5,531,746 concerns a polyaxial lateral mass locking screw plate assembly for immobilization of vertebral bones, via fixation to surfaces thereof.

FR 2 861 980 AI discloses a system, designed to join a number of vertebrae together, consisting of an implant with one or more holes for anchoring screws and locking elements. The locking elements are in the form of elastically-deformable rings with surfaces that are able to co-operate with the threads of the anchoring screws and external ribs that clip into the holes.

US2005/0043736 A1 concerns a device for performing osteosynthesis includes a fixation element, such as a bone plate having a bottom surface designed to bear against the bone, a top surface, and at least one through hole extending from the bottom surface to the top surface, the though hole having a central axis and configured and dimensioned toreceive a multiaxially pivotal insert or bushing for a bone screw.

US 6,235,033 relates to a bone fixation assembly for securing a fixation device, such as a bone plate, to bone. The assembly includes the fixation device, a bushing, a fastening screw, and a locking screw.

WO 2007/014279 A2 relates to a bone fixation assemblyl that can provide polyaxial fixation.

US 6,030,389 relates to a spinal plate system and method for xiation of the human spine. The system includes a plate, a coupling member and a locking system for substantially locking the coupling member in a desired position.

The field of orthopedics has included countless advances in the design of implants for internal fixation. The present invention provides an advance in the design of an assembly which allows a surgeon to choose whether to use a locking fixator including for example, a screw, or peg locked at a predetermined angle or to use a variable locking fixator inserted through a stabilizer, such as a plate, anchor or cage, at a variable angle in order to best capture a bone or bone segment with the fixator. The angle can subsequently be locked to fix the bone or bone segment relative to the plate, or to fix the plate relative to the bone or bone segment. The invention allows for at least about 25° and more preferably 30° of angulation relative to a longitudinal axis of the opening that the fixator is inserted through.

There are numerous applications which can benefit from such a mechanism. Specific examples include use in the small bones, such as the metacarpals and carpals, and the metatarsals and tarsals, although it is understood, that the mechanism can also be of great use in other areas of the body, including the long bones, the pelvis and the spine.

### SUMMARY OF THE INVENTION According to the present invention, an orthopedic implant according to claim 1 and amethod according to claim 10 are provided. Prefered embodiments of the invention are set forth in the dependent claims.

The assembly of the present invention includes a convexly rounded, and optionally spherical locking ring that is seated in the concavely rounded corresponding opening in the implant. The locking insert is deformable relative to the opening in which it seats. In addition, it is preferable that the opening includes high friction deformation means, such as a series of internal threads that bite into the locking insert so as to deform the ring and bind it into position relative to the plate. This further provides the advantage that the locking insert can be used in a threaded locking opening, allowing the surgeon the option of using a threaded locking screw to lock the screw in the plate at a selected angle or to use the present assembly to select a different angle.

In particular, the fixator, may be a screw or peg, which includes a flange that extends radially outward and around the head of the screw or peg which compliments the profile of the locking ring and provides a surface that mates with a bevel in the opening of the locking ring. The head includes a slight taper on the exterior surface below the flange. The lócking ring seats below the flange on the head and engages the tapered surface. The portion of the screw head which is axially proximal to the flange also compliments the locking ring so as to form a non-obtrusive or low profile in the screw hole. As the fixator is inserted into the plate, the flange presses the locking ring downward into a deformable contact with the internal threads in order to cause a friction fit of the locking insert in the opening and to lock the screw or peg at the desired angle. The ring and the screw are essentially one unit as delivered to the user. The ring is assembled onto the screw so as to remain integral to the screw head. Alternatively, the ring can be assembled into the opening, and the screw can be inserted through the ring to assemble the variable locking assembly. Preferred method of assembly is the use of a Morse taper (approx 6-7 degrees) on the screw head and the ring interior to fix the two components together. The ring and screw can be integrated together by mechanical means including the interference fit previously mentioned, by other interlocking means including for example, locking flanges or friction surfaces, or chemical bonding can be used, including adhesives. Additionally, the ring will preferably have a geometry that fits onto the screw head to prevent rotation of the ring with respect to the screw during insertion. The ring could be either machined and assembled or over-molded onto the screw.

### BRIEF DESCRIPTION OF THE DRAWINGS

Figure 1 is a cross section of a plate illustrating embodiment of the variable axis locking mechanism assembly in accordance with the present invention;
Figure 2 is a perspective view from the top and the side of a locking insert of the present invention;
Figure 3 is a side view of the locking insert of Figure 2; and
Figure 4 is a side view of a screw that can be used as part of the locking mechanism assembly of the present invention.

### DETAILED DESCRIPTION OF THE INVENTION

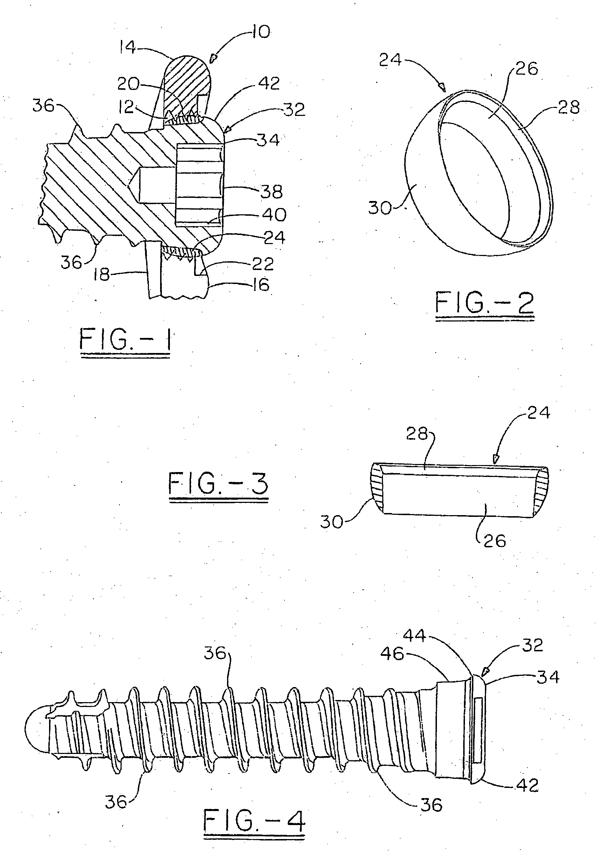

Figure 1 shows a variable axis locking mechanism assembly 10 in an opening 12 in the plate 14 which is representational of any number of plate designs that can use the present invention. The plate has a top surface 16 and a bottom surface 18 (which is the surface that opposes, if not engages the bone or bone segments for which the plate is used.) The plate includes an opening 12 that extends from the top surface 16 though to the bottom surface 18 which includes internal threads 20 and a counterbore or recess 22 which provides a seat for the use of a drill guide and which provides a zero point in the inspection of the taper depth during manufacture. While the opening is threaded, it is shaped for a threaded member having a taper in both the major and the minor diameter.

The locking ring 24 has an internal opening 26 with a bevel 28 that leads into the opening. The external surface 30 of the locking ring is rounded and together with a rounded shoulder of the fixator 32 forms a smoothly rounded profile to accommodate a variable angle and which has no sharply projecting edges to would irritate soft tissue after implantation. The fixator 32 is in this case a screw having a head 34 and distal threads 36. The head includes a top surface 38 with an internal torque driving recess 40. The top surface 38 includes a rounded shoulder 42 which is under cut to form a concentric flange 44. The flange joins a side wall 46 having a circular cross section, but which tapers toward the distal end of the screw. The taper is from about 5° to about 15°, and preferably about 6° to about 10°. The screw includes a necked area which joins the head to a threaded area which includes a cancellous bone screw 48. The screw head can also include other means which will couple the locking insert and the screw head, including for example, a series of concentric flanges, knurling, or threads.

The locking ring shaped insert, having smoothly rounded exterior walls 30, which optionally can include a higher friction surface as is created by knurling, milling or otherwise roughening or texturing the surface. The locking ring is made from a material, which is relatively deformable compared to the plate in which it seats. Thus, the insert will deform rather than splinter as it engages the seat. For example, the locking ring may be made of a biocompatible polymer or ceramic, such as as PEEK or UHMWPE (ultra high molecular weight polyethylene), or a softer metal, such as stainless or commercially pure titanium in a titanium alloy plate.

## Claims

1. A variable angle locking mechanism assembly for an-orthopedic implant comprsing:
the plate (14) made of a first material and having a through opening (12) having a longitudinal axis, the opening including internal threads;
a locking ring shaped insert (24), which is a continuous ring, made of a second material which is more deformable than the first material, and the locking ring shaped insert having an internal opening (26) defining a longitudinal axis and having a convexly rounded side wall and sized to fit in the through opening (12) in the plate; and
a fastener (32) that is adapted to extend through and engage the internal opening of the locking ring shaped insert (24) and having a head (34) that includes an undercut (42) to form a helical flange member (44) which mates with the locking ring shaped insert to selectively lock the insert at an angle in the through opening relative to the longitudinal axis as the side wall of the insert engages the internal threads of the through opening, so as to deform the ring and bind it into position relative to the plate, wherein the flange member joins a side wall (46) having a circular cross section tapering towards the distal end of the fastener, wherein the taper is from about 5° to about 15°, wherein the locking ring shaped insert is adapted to, when assembled into the through opening (12) in the plate, to allow the fastener to be locked at an angle selected from a plurality of angles which allows for at least 25° of angulation relative to the longitudinal axis of the through opening (12) and which has an interference fit with the fastener and wherein the interior surface of the locking ring shaped insert has a taper.

2. The variable angle locking mechanism assembly as set forth in claim 1, wherein the fastener (32) is a peg or a screw having a head (34) having concentric flanges, knurling or threads.

3. The variable angle locking mechanism assembly as set forth in claim 2, wherein the locking insert (24) includes a proximal bevel (28) and the head (34) of the fastener includes a concentric flange (44) that engages the bevel (28) of the locking insert and further where the proximal side of the flange is rounded in the proximal direction such that the locking insert compliments the head of the fastener.

4. The variable angle locking mechanism assembly as set forth in claim 1, wherein the locking ring shaped insert (24) is integral to the fastener (32).

5. The variable angle locking mechanism assembly as set forth in claim 1, wherein the internal threads bite into the locking insert.

6. The variable angle locking mechanism assembly as set forth in claim 1, wherein the fastener (32) is a peg or a screw.

7. The variable angle locking assembly as set forth in claim 1, wherein the first material is metal and the second material is not metal.

8. The variable angle locking assembly as set forth in claim 7, wherein the second material is a ceramic or a polymer.

9. The variable angle locking assembly as set forth in claim 1, wherein the first material is an alloy of titanium and the second material commercially pure titanium.

## Patentansprüche

1. Arretiermechanismusanordnung mit variablem Winkel für ein orthopädisches Implantat, umfassend:
die Platte (14), die aus einem ersten Material hergestellt ist und die eine durchgehende Öffnung (12) aufweist, welche eine Längsachse hat, wobei die Öffnung Innengewinde umfasst;
einen ringförmigen Arretiereinsatz (24), der ein durchgehender Ring ist, welcher aus einem zweiten Material hergestellt ist, das verformbarer als das erste Material ist, und wobei der ringförmige Arretiereinsatz eine innenliegende Öffnung (26), die eine Längsachse definiert, und eine konvex gerundete Seitenwand aufweist und er in seiner Größe so bemessen ist, dass er in die durchgehende Öffnung (12) in der Platte hineinpasst;
ein Befestigungselement (32), das geeignet ist sich durch die innenliegende Öffnung des ringförmigen Arretiereinsatzes (24) zu erstrecken und in diese einzugreifen und das einen Kopf (34) aufweist, der einen Hinterschnitt (42) umfasst, um ein spiralförmiges Flanschelement (44) zu bilden, das mit dem ringförmigen Arretiereinsatz zusammenpasst, um den Einsatz selektiv in einem Winkel relativ zu der Längsachse in der durchgehenden Öffnung zu arretieren, weil die Seitenwand des Einsatzes in die Innengewinde der durchgehenden Öffnung eingreift, sodass der Ring verformt wird und er relativ zu der Platte in Position gehalten wird, wobei das Flanschelement an eine Seitenwand (46) angrenzt, die einen kreisförmigen Querschnitt hat, der sich hin zum distalen Ende des Befestigungselements verjüngt, wobei die Verjüngung etwa 5° bis etwa 15° beträgt, wobei der ringförmige Arretiereinsatz geeignet ist, wenn er in der durchgehenden Öffnung (12) in der Platte angeordnet ist, dem Befestigungselement zu ermöglichen, in einem Winkel, der aus einer Vielzahl an Winkel ausgewählt ist, arretiert zu werden, was eine Angulation von mindestens 25° relativ zu der Längsachse der durchgehenden Öffnung (12) ermöglicht und was eine Presspassung mit dem Befestigungselement aufweist, und wobei die innenliegende Oberfläche von dem ringförmigen Arretiereinsatz eine Verjüngung aufweist.

2. Arretiermechanismusanordnung mit variablem Winkel nach Anspruch 1, wobei das Befestigungselement (32) ein Stift oder eine Schraube ist, der oder die einen Kopf aufweist (34), der konzentrische Flansche, eine Rändelung oder Gewinde hat.

3. Arretiermechanismusanordnung mit variablem Winkel nach Anspruch 2, wobei der Arretiereinsatz (24) eine proximale Schräge (28) umfasst und der Kopf (34) des Befestigungselements einen konzentrischen Flansch (44) umfasst, der in die Schräge (28) des Arretiereinsatzes und ferner dort eingreift, wo die proximale Seite des Flansches in der proximalen Richtung gerundet ist, sodass der Arretiereinsatz den Kopf des Befestigungselements komplementiert.

4. Arretiermechanismusanordnung mit variablem Winkel nach Anspruch 1, wobei der ringförmige Arretiereinsatz (24) ein integraler Bestandteil des *Befestigungselements (32) ist.

5. Arretiermechanismusanordnung mit variablem Winkel nach Anspruch 1, wobei die Innengewinde in den Arretiereinsatz einschneiden.

6. Arretiermechanismusanordnung mit variablem Winkel nach Anspruch 1, wobei das Befestigungselement (32) ein Stift oder eine Schraube ist.

7. Arretiermechanismusanordnung mit variablem Winkel nach Anspruch 1, wobei das erste Material Metall und das zweite Material kein Metall ist.

8. Arretiermechanismusanordnung mit variablem Winkel nach Anspruch 7, wobei das zweite Material ein keramisches Material oder ein Polymer ist.

9. Arretiermechanismusanordnung mit variablem Winkel nach Anspruch 1, wobei das erste Material eine Titanlegierung und das zweite Material handelsübliches reines Titan ist.

## Revendications

1. Ensemble de mécanisme de verrouillage à angle variable pour un implant orthopédique comprenant :
une plaque (14) réalisée avec un premier matériau et ayant une ouverture débouchante (12) ayant un axe longitudinal, l'ouverture comprenant des filetages internes ;
un insert en forme d'anneau de verrouillage (24), qui est un anneau continu, réalisé avec un second matériau qui est plus déformable que le premier matériau et l'insert en forme d'anneau de verrouillage ayant une ouverture interne (26) définissant un axe longitudinal et ayant une paroi latérale arrondie de manière convexe et dimensionnée pour s'adapter dans l'ouverture débouchante (12) dans la plaque ; et
une fixation (32) qui est adaptée pour s'étendre à travers et mettre en prise l'ouverture interne de l'insert en forme d'anneau de verrouillage (24) et ayant une tête (34) qui comprend un dégagement (42) pour former un élément de bride hélicoïdal (44) qui se couple avec l'insert en forme d'anneau de verrouillage pour verrouiller sélectivement l'insert à un angle dans l'ouverture débouchante par rapport à l'axe longitudinal lorsque la paroi latérale de l'insert met en prise les filetages internes de l'ouverture débouchante, afin de déformer l'anneau et l'attacher en position par rapport à la plaque, dans lequel l'élément de bride est raccordé à une paroi latérale (46) ayant une section transversale circulaire se rétrécissant progressivement vers l'extrémité distale de la fixation, dans lequel la conicité est d'environ 5° à environ 15°, dans lequel l'insert en forme d'anneau de verrouillage est adapté, lorsqu'il est assemblé à l'ouverture débouchante (12) dans la plaque, pour permettre de verrouiller la fixation à un angle choisi parmi une pluralité d'angles qui permet au moins 25° d'angulation par rapport à l'axe longitudinal de l'ouverture débouchante (12) et qui a un ajustement avec serrage avec la fixation et dans lequel la surface intérieure de l'insert en forme d'anneau de verrouillage a une conicité.

2. Ensemble de mécanisme de verrouillage à angle variable selon la revendication 1, dans lequel la fixation (32) est une goupille ou une vis ayant une tête (34) ayant des brides concentriques, un moletage ou des filetages.

3. Ensemble de mécanisme de verrouillage à angle variable selon la revendication 2, dans lequel l'insert de verrouillage (24) comprend un biseau proximal (28) et la tête (34) de la fixation comprend une bride concentrique (44) qui met en prise le biseau (28) de l'insert de verrouillage et en outre où le côté proximal de la bride est arrondi dans la direction proximale de sorte que l'insert de verrouillage complète la tête de la fixation.

4. Ensemble de mécanisme de verrouillage à angle variable selon la revendication 1, dans lequel l'insert en forme d'anneau de verrouillage (24) est solidaire de la fixation (32).

5. Ensemble de mécanisme de verrouillage à angle variable selon la revendication 1, dans lequel les filetages internes mordent dans l'insert de verrouillage.

6. Ensemble de mécanisme de verrouillage à angle variable selon la revendication 1, dans lequel la fixation (32) est une goupille ou une vis.

7. Ensemble de verrouillage à angle variable selon la revendication 1, dans lequel le premier matériau est métallique et le second matériau n'est pas métallique.

8. Ensemble de verrouillage à angle variable selon la revendication 7, dans lequel le second matériau est une céramique ou un polymère.

9. Ensemble de verrouillage à angle variable selon la revendication 1, dans lequel le premier matériau est un alliage de titane et le second matériau est du titane techniquement pur.
